# EUROPEAN PATENT SPECIFICATION

(11) **EP 3 047 809 B1**
(45) Date of publication and mention of the grant of the patent: **13.04.2022**
(21) Application number: 16152465.7
(22) Date of filing: 22.01.2016
(51) Int. Cl.: A61B 17/225, A61B 34/20, A61B 8/00

(54) **EXTRACORPOREAL SHOCK WAVE LITHOTRIPSY SYSTEM HAVING OFF-LINE ULTRASOUND LOCALIZATION**
EXTRAKORPORALES STOSSWELLENLITHOTRIPSIESYSTEM MIT OFFLINE-ULTRASCHALLORTUNG
SYSTÈME DE LITHOTRITIE PAR ONDES DE CHOC EXTRACORPORELLES À LOCALISATION ULTRASONORE HORS LIGNE

(30) Priority: 23.01.2015 EP 15152381
(43) Date of publication of application: 27.07.2016
(73) Proprietor: STORZ MEDICAL AG, 8274 Tägerwilen (CH)
(72) Inventor: Heine, Gerold, 8274 Tägerwilen (CH); Hagelauer, Ulrich, 78464 Konstanz (DE); Novak, Pavel, 8234 Stetten (CH); Gleibe, Olaf, 8280 Kreuzlingen (CH); Keil, Christina, 8274 Tägerwilen (CH); Görner, Georg, 8597 Landschlacht (CH); Koch, Axel, 8274 Tägerwilen (CH); Stumm, Alexander, 88048 Kluftern (DE); Kühl, Arvid, 8274 Tägerwilen (CH)
(74) Representative: Lohr, Jöstingmeier & Partner

(56) References cited:
- WO-A2-2015/092664
- DE-A1-102006 060 070
- DE-C1- 10 206 193
- US-A- 4 896 673
- US-A- 5 295 483
- US-A1- 2003 149 352
- US-A1- 2006 058 616
- US-A1- 2011 166 450
- US-A1- 2012 275 645
- US-A1- 2013 218 024
- US-B1- 6 216 029
- US-B1- 6 546 279
- Po-Wei Hsu ET AL: "Freehand 3D Ultrasound Calibration: A Review" In: "Advanced Imaging in Biology and Medicine", 1 January 2009 (2009-01-01), Springer Berlin Heidelberg, Berlin, Heidelberg, XP055193980, ISBN: 978-3-54-068992-8 pages 47-84, DOI: 10.1007/978-3-540-68993-5_3, * pages 11-13,21; figure 11 *
- TROBAUGH J W ET AL: "Frameless stereotactic ultrasonography: Method and applications", COMPUTERIZED MEDICAL IMAGING AND GRAPHICS, PERGAMON PRESS, NEW YORK, NY, US, vol. 18, no. 4, 1 July 1994 (1994-07-01), pages 235-246, XP022916194, ISSN: 0895-6111, DOI: 10.1016/0895-6111(94)90048-5 [retrieved on 1994-07-01]
- TOMÉ WOLFGANG A ET AL: "Commissioning and quality assurance of an optically guided three-dimensional ultrasound target localization system for radiotherapy", MEDICAL PHYSICS, AIP, MELVILLE, NY, US, vol. 29, no. 8, 1 August 2002 (2002-08-01) , pages 1781-1788, XP012011880, ISSN: 0094-2405, DOI: 10.1118/1.1494835
- MERCIER L ET AL: "A review of calibration techniques for freehand 3-D ultrasound systems", ULTRASOUND IN MEDICINE AND BIOLOGY, NEW YORK, NY, US, vol. 31, no. 2, 1 February 2005 (2005-02-01), pages 143-165, XP027605560, ISSN: 0301-5629 [retrieved on 2005-02-01]

## Description

### Field of the invention

The invention relates to an extracorporeal shock wave lithotripsy system for non-invasive treatment of stones, like e.g. kidney stones, urinary stones, gallstones or other calculi within a mammal's body using acoustic pulses. The extracorporeal shock wave lithotripsy system uses off-line ultrasound localization.

### Description of the related art

A device and a method for localizing stones and for targeting a shock wave source of a lithotripter is disclosed in DE 198 41 951 A1. It uses X-ray imaging and generates a pseudo 3D image on a screen to simplify targeting. To avoid x-ray exposure, in-line ultrasound systems have been developed. Here, a diagnostic ultrasound transducer is arranged in a line with the therapeutic shock wave source. An off-line ultrasound localization, using a handheld, freely positionable ultrasound transducer is more flexible and allows better images. A further targeting method is disclosed in EP 2 340 781 A1 (also published as US 2011/0166450 A1). Here a shockwave generator, a patient table and an ultrasound targeting device are provided with optical markers for tracking with a pair of infrared cameras. The precision and reliability of the tracking system determine the total system precision. A verification may be done by using an X-ray system.

US 5,817,105 and US 5,954,648 disclose image-guided surgery systems. US 5,828,770, US 5,923,417, and US 6,061,644 disclose systems for determining the spatial position and orientation of a body.

US 7,945,311 B2 and US 7,137,712 B2 discloses a reflector system for determining position. US 2006/0058616 A1 discloses an interactive computer-assisted surgery system and method. US 6,216,029 B1 discloses a method for free-hand directing of a needle towards a target located in a body volume.

### Summary

A problem to be solved by the invention is to provide an extracorporeal shock wave lithotripsy system with off-line ultrasound localization which allows for automatic recognition of the arrangement, in particular the position and the orientation of the system components, in particular the ultrasound transducer, the therapy source and the patient table.

A further problem to be solved by the invention is to provide an easy means to verify the precision and the functionality of such a system at any time.

Yet a further problem to be solved by the invention is to provide such a system which can verify that tracking of the system components is working correctly without using other targeting means such as e.g. in-line ultrasound or X-ray.

An embodiment provides a system calibration phantom for use with an extracorporeal shock wave lithotripsy system with off-line ultrasound localization whose functionality can be verified without other targeting means such as e.g. in-line ultrasound or X-ray. This calibration phantom should be easy to use and have an improved precision as compared to phantoms of the prior art.

Furthermore, operation of the extracorporeal shock wave lithotripsy system should be simplified and it should be possible to follow the treatment in real-time.

The extracorporeal shock wave lithotripsy system should also be able to move a stone which was detected by the ultrasound system automatically towards a focal zone of a therapy source or vice versa. Additionally or alternatively such a movement should be executable manually. In this case, the system should be adapted to help an operator to achieve this goal.

Solutions of the problem are described in the independent claim 1. The dependent claims relate to further improvements of the invention.

In accordance with the present invention, an extracorporeal shock wave lithotripsy system comprises a patient table for supporting a patient, an ultrasound transducer for examining and imaging a body part of a patient, a therapy source for treating the body part of the patient and at least one targeting instrument. The at least one targeting instrument comprises a localizing instrument for measuring positions of the table, the therapy source or imaging systems. Preferably, the localizing instrument comprises a camera for capturing images of visual references. The camera may be an infrared (IR) camera system, e.g. an image-guided surgery system as disclosed in US 5,817,105 or US 5,954,648. The camera may further use a system for determining the spatial position and orientation of a body as disclosed in US 5,828,770, US 5,923,417, and US 6,061,644. The targeting instruments may also comprise a laser scanner.

The camera may also be working in other frequency ranges of light, like e.g. visible or UV. The camera may e.g. be mounted adjustably on a therapy source, or on or at the patient table.

A visual reference comprises four visual reference markers. A visual reference marker may e.g. be any device of which the position and orientation may be detected by the camera. A visual reference marker may e.g. be a reflector system for determining position as disclosed in US 7,945,311 B2, US 7,137,712 B2. Such reflector systems are used with image-guided surgery systems as disclosed in the patents cited above.

Each visual reference marker may comprise three such lenses, preferably four such lenses. Using three lenses, all six degrees of freedom (6DOF), i.e. three translational degrees of freedom and three rotational degrees of freedom, may be measured. Compared with the case of three lenses, the case of four lenses has the advantage of increased accuracy.

The camera is arranged on a trolley which is freely movable on the floor. Additionally the camera may be fixed in space, e.g. by fixing the trolley. The camera may e.g. also be arranged on or at the therapy source or on or at the patient table.

The extracorporeal shock wave lithotripsy system further comprises a patient table having a first mounting rail along a first longitudinal side of the patient table and a second mounting rail along a second longitudinal side of the patient table. The second longitudinal side is opposite to the first longitudinal side and a first visual reference is arranged on the first mounting rail or the first longitudinal side and a second visual reference is arranged on the second mounting rail or the second longitudinal side. By providing visual reference on both sides of the patient table, i.e. on the right hand and the left hand side of a patient, for automated positioning of the system, the system can recognize the orientation of the patient table with respect to a visual reference of a therapy source.

The patient preferably lies on his back while an operator examines his body, e.g. abdomen, using an ultrasound transducer from the top or the side. The patient table may be moved by a translation stage or some other motor actuator. According to a different embodiment, the patient table comprises only one mounting rail along a longitudinal side of the patient table and only one visual reference is arranged on the mounting rail or the longitudinal side. In this case for automated positioning of the system, if the patient table is moved to a predefined direction, e.g. clockwise or counterclockwise, it is possible to determine the orientation of the patient table by tracking the movement of the ultrasound transducer.

The extracorporeal shock wave lithotripsy system further comprises an ultrasound transducer for examining and imaging a body part of the patient. The body part of the patient may comprise stones, like e.g. kidney stones, gallstones, or other calculi which formed through lithiasis. For localization of stones within a patient's body an ultrasound localization system as above mentioned is used. The ultrasound transducer comprises a third visual reference.

An operator may use the ultrasound device the same way as in normal diagnostic ultrasound probe. Free angulation and positioning of the transducer allows him to find a good entrance window. If a good image slice of the kidney and the stone is reached by the operator, the ultrasound transducer may be fixed in its position.

Preferably, the ultrasound image should be calibrated. This means that the relative position of any pixel of the ultrasound image may be determined with respect to an ultrasound transducer head. It is further preferred that the position and orientation of the ultrasound plane and/or the ultrasound transducer is known with respect to the third visual reference, the third visual reference being arranged somewhere on the ultrasound transducer or on an ultrasound transducer mount. The ultrasound image may e.g. be calibrated by the calibration phantom described elsewhere in this description.

Additionally or alternatively to the ultrasound transducer, a C-arm having an X-ray source may be provided. By evaluating two X-ray images taken a sufficiently different angular positons, e.g. larger than 20°, the position of the stone inside the patient's body may be determined. The C-arm comprises a sixth visual reference for this purpose.

The extracorporeal shock wave lithotripsy system further comprises a therapy source for treating the body part of the patient. The therapy source comprises a fourth visual reference and a shock wave source which comprises a focal zone for destroying the stone inside the patient's body. Preferably the position, extent and orientation of the focal zone is known relative to the fourth visual reference of the therapy source.

Preferably, the visual reference of the therapy source is used as global reference for the camera. It may be fixed with respect to the focal zone of the therapy source and may be attached to a supporting structure attached to a column of the therapy source, on a supporting structure attached to the therapy source or on an stationary X-ray or mobile C-arm (if provided). Alternatively, the visual reference may be fixed on a pivot joint of a reference arm. In case there is a direct line of sight from the camera to the therapy source, the visual reference of the therapy source may be directly fixed to the therapy source. The therapy source may be moved by a translation stage or some other motor actuator.

Preferably, the grabbed and processed ultrasound image is correlated by time code to the acquired position of the ultrasound transducer. If an unknown delay occurs there might be a deviation between calculated and real time stone position.

Preferably, the ultrasound image is acquired in good image quality, most preferable as a digital video signal of the ultrasound device. The ultrasound image may be calibrated, so that the dimensions (pixels) are known. Furthermore the image should not be mirrored or zoomed. Preferably, this may be monitored by a software, preferably the GUI, and in case the image is mirrored or zoomed, an alert or an error message is displayed by the software. To prevent misadjusted images a control overlay may be used to give an optical indication if the grabbed ultrasound-image is aligned for "stone acquisition".

In order to give the operator a visual verification that the stone is in the therapy zone, a focus overlay in the real size is preferred. The size of the focal or therapy zone may be changed by altering the energy level of the shock wave source. If the size of the display of the ultrasound image is changed, the size of the focal zone should be changed accordingly.

Preferably, the set penetration depth of the ultrasound transducer should be recognized by the software, e.g. a GUI described below. By zooming the ultrasound image, the size of the displayed therapy zone may also change. Depending on the height of the ultrasound scan plane within the therapy zone, the diameter of the therapy zone may be available in several steps.

If the therapy zone is below or above the ultrasound scan plane, the software may show superimposed arrows on the ultrasound image, wherein the arrows show the direction into which the therapy zone should be moved. If the therapy zone is out of the table range, the software, e.g. the GUI described below, should show an error message.

Preferably, the position of the stone in space relative to the ultrasound transducer is known and the vector from the position of the stone to the therapy zone can be calculated. The vector may be used to position the patient table such that the stone is positioned inside the focal zone of the therapy source. For automatic positioning of the stone to the focal zone, the vector for the required table movement may be calculated. Prerequisite is a marked stone in a frozen ultrasound image with known position in space of the ultrasound transducer and image data input interface (frame grabber, video input). The camera may recognize the position of each marker (ultrasound transducer and therapy head). A difference vector (Δx, Δy, Δz) between the marked stone and the focal zone may be calculated by the measurement and evaluation system, e.g. a computer system, and be forwarded to the measurement and evaluation system of the extracorporeal shock wave lithotripsy system for automatic stone positioning. Automatic stone positioning could be done by moving the patient table towards the focal zone or by moving the therapy source with respect to the patient table towards the stone.

Furthermore, manual ultrasound stone positioning may be provided with optimized, workflow oriented GUI providing user guidance. 3D visualization may be provided for orientation in space, a virtual reality scenario might help a user in case of manual positioning. Here, the correct positioning buttons may be highlighted until the difference vector is minimal or zero.

The extracorporeal shock wave lithotripsy system further comprises a displacing means for changing a relative position of the patient table and the therapy source. This may e.g. be realized by two translation stages, wherein one translation stage is coupled to the patient table and another one translation stage is coupled to the therapy source. The relative position and orientation of the two translation stages should preferably be known.

The extracorporeal shock wave lithotripsy system further comprises a measurement and evaluation system, like e.g. a computer. The measurement and evaluation system is connected to the ultrasound transducer, the camera, the therapy source, and the displacing means. The measurement and evaluation system may e.g. also used to control at least one, preferably all, of those components. The measurement and evaluation system inter alia is adapted to evaluate relative positions between at least two, preferably all, of the ultrasound transducer, the therapy source, the first mounting rail and the second mounting rail. The measurement and evaluation system may e.g. be arranged on the trolley, preferably together with the display.

The extracorporeal shock wave lithotripsy system further comprises a display connected to the measurement and evaluation system for displaying a relative position of at least the body part and a position of the focal zone of the therapy source. The display is connected to the measurement and evaluation system which collects data from the camera and evaluates these data. As the camera may detect the position of each visual reference, positional data of the patient table, the ultrasound transducer and the therapy source may be acquired. After evaluation of the positional data (preferably including position and orientation) of the first mounting rail, or the second mounting rail, the ultrasound transducer, and the therapy source all or part of these data may be fed to the display for displaying a relative position of at least two of, preferably all of, these visual references.

The display may e.g. be arranged or mounted on the trolley or on a separate table using a monitor arm. The display may e.g. be arranged or mounted to a monitor arm.

The ultrasound transducer may have at least two different positions at the patient table. For treatment of the patient the ultrasound transducer may be mounted in an operating position, where it is e.g. mountable to the patient table, the first or the second mounting rail. The ultrasound transducer may e.g. be mountable to the patient table using a flexible arm. The flexible arm may e.g. be an adjustable articulated arm. During treatment of the patient the ultrasound transducer is freely adjustable in space for adjusting the position and orientation of the ultrasound transducer and lockable at a desired position and orientation. This has the advantage that an ultrasound image of the patient's body may be fixed relative to the patient table on which the patient's body rests. This avoids disturbance and inaccuracy of the tracking system as compared to systems which are mobile in space and therefore are not fixed relative to the patient table.

By mechanically connecting the ultrasound transducer to the patient table, the orientation of the patient table may be detected by moving the table into a predetermined direction.

When the ultrasound transducer is not used for treatment of the patient, it may e.g. be in a non-operating position, where it is mounted e.g. to the trolley.

Each of the first visual reference, the second visual reference, the third visual reference and the fourth visual reference may e.g. comprise three visual references, preferably four visual references. Using three lenses, all six degrees of freedom (6DOF), i.e. three translational degrees of freedom and three rotational degrees of freedom, may be measured. Compared with the case of three lenses, the case of four lenses has the advantage of increased accuracy.

The therapy source may e.g. comprise a reference arm which is rotatably arrangeable with respect to the therapy source. The reference arm preferably comprises the fourth visual reference. The feature that the reference arm is rotatably arrangeable with respect to the therapy source has the advantage that the reference arm could be swiveled from the first longitudinal side of the patient table to the second longitudinal side of the patient table. Another advantage is that this can save the reference arm from damage because at a touch of some other object, the reference arm will move away from this other object.

Preferably, the reference arm is arranged within an opening which is normally used for inline tracking.

In an alternative embodiment, the visual reference of the therapy source may e.g. be mounted to a different location, for example to an X-ray C-arm.

Preferably the position of the reference arm is fixed relative to therapy source. This has the advantage that the position of the reference are is known with respect to the therapy source.

The displacing means is adapted to change a relative position of the patient table and the therapy source such that a position of the stone falls within the focal zone. The changing or actuating of the relative position may preferably be executed automatically or manually. This execution or actuation may e.g. be done by the measurement and evaluation system.

The camera is preferably is in a position ensuring a line of sight to the visual references necessary for tracking the ultrasound transducer and therapy source, i.e. the third and fourth visual references and preferably also the first mounting rail and/or the second mounting rail, i.e. the first and second visual references. If there is an X-ray C-arm, it is preferred that there is also a visual reference on the C-arm which preferably should also be seen by the camera. It might be useful that the camera position is adjustable in a certain range for optimizing the line of sight for scanning different body parts on the left or right side of the patient's body. The measurement volume of the optical tracking system, i.e. the camera, should cover the visual references on the therapy source and the ultrasound transducer, independent of the examined side of the patient.

The measurement and evaluation system and/or the display comprises a graphical user interface (GUI). The purpose of the graphical user interface is inter alia to display a positional relationship, in particular an overlay, of at least the focal zone of the therapy source and the ultrasound plane generated by the ultrasound transducer. In this overlay other components of the extracorporeal shock wave lithotripsy system may also be displayed or overlaid.

If the actuation of the relative position is executed manually, the GUI may indicate e.g. on the display in which direction a user should actuate a body to be actuated. The body to be actuated may e.g. be the patient table or the therapy source. If the therapy source, i.e. the focal zone, is actuated, the GUI indicates the direction into which the focal zone should be moved in order to be overlapped with the stone. If the patient table, i.e. the stone, is actuated, the GUI indicates the direction into which the stone should be moved in order to be overlapped with the focal zone of the therapy source.

When the moving is done using cursor keys, the GUI may highlight the cursor key which should be pressed to achieve the aim.

The GUI may e.g. also be adapted to show a view of at least one position of the first visual reference, the second visual reference, the third visual reference and the fourth visual reference within the tracking volume of the camera. The view might be a top, side or frontal view. This allows easy adjustment of the camera.

Preferably, the graphical user interface is adapted to show a detection region of at least one of the first visual reference, the second visual reference, the third visual reference and the fourth visual reference.

In accordance with the present invention, the graphical user interface is adapted to show a detection region of the third visual reference which is arranged on the ultrasound transducer and of the fourth visual reference which is arranged on the therapy source. Detection region means the region wherein a visual reference may be detected by the camera.

A status of the detection region is preferably indicated in the GUI. A status might e.g. be that the visual reference is within or outside of the detection region. If the respective visual reference is outside of the detection region of the camera, an alert may e.g. be indicated by the GUI, e.g. by showing a red frame and a message that the respective visual reference is outside the detection region.

Preferably, the graphical user interface is adapted to show the graphical position of the ultrasound plane fixed while the graphical position of the focal zone is movable relative to the graphical position of the ultrasound plane. Additionally or alternatively the graphical user interface may e.g. be adapted to show the graphical position of the focal zone fixed while the graphical position of the ultrasound plane is movable relative to the graphical position of the ultrasound plane.

Preferably, the GUI is adapted such that for marking of some features of the ultrasound image, e.g. for marking of the stone inside the patient's body, the display or the image displayed on the GUI may e.g. be frozen and the position of a special feature may be marked.

The coordinates of the marked feature may be forwarded to the therapy source. Then the patient table or the therapy source may be moved automatically such that the stone is within the focal zone, which may also be called therapy zone.

A wireless (e.g. Bluetooth or voice controlled) connected freeze switch on the ultrasound transducer may be provided to help the operator to freeze an ultrasound image without turning away from the treatment area. Alternatively or additionally such a freeze switch may be arranged on the probe or on a foot pedal.

It is further preferred to have a freeze button on the ultrasound transducer. The operator may easily locate and freeze the optimal slice of the kidney by pressing the button.

It is further preferred, that at least one means for stone marking is provided to be performed on the frozen ultrasound image by means of a pointing device of a computer. Preferably, a touch screen is used. A mark like circle, square or cross-hairs may indicate a selected area. A fine adjustment of this mark by buttons may be possible as well.

Preferably the graphical user interface is adapted to show positions of at least two of the first reference, the second reference, the third reference and the fourth reference within a tracking volume of the camera. Preferably the graphical user interface is adapted to show all positions of the first reference, the second reference, the third reference and the fourth reference within a tracking volume of the camera.

The graphical representation of any of the visual references may e.g. be a front or a plan view.

If a visual reference is out of the tracking volume, the GUI may e.g. display an alert by e.g. a red frame. In this case the symbol for the focal zone is preferably not displayed any more.

Knowing the position of the ultrasound probe alone may not be adequate to determine the positions of the acquired 2D images. The relationship between these two coordinate frames may be calculated through the process of ultrasound probe calibration. The calibration procedure needs to be fast, robust and easy to perform with minimal human interaction. The ultrasound image has to be calibrated in order to insure proper measurements. Therefore a calibration phantom may be provided.

An embodiment, not part of the invention relates to a calibration phantom for an ultrasound transducer for examining a body part of a patient.

The calibration phantom comprises a hollow body having a location reference for the ultrasound transducer. The hollow body might be closed at a bottom side. In that case the body might be a receptacle into which a fluid may be filled. Alternatively, the hollow body might be open at a bottom side. In this case the fluid might be in another receptacle required for holding the fluid. The fluid is preferably water or some other liquid which has similar properties as the tissue of human beings or other mammals. Here, the term properties preferably refers to the acoustic properties of the fluid.

Preferably, the calibration phantom or the hollow body approximately has a cuboidal shape.

The calibration phantom further comprises a plurality of fibers or threads comprising a material visible to the ultrasound transducer. Each fiber is preferably fixed within the hollow body, preferably at two opposing walls of the hollow body.

According to a first embodiment of the calibration phantom, at least one pair of crossed threads is provided inside the phantom. It is further preferred that the calibration phantom comprises at least three pairs of threads fixed within the hollow body, wherein each pair of threads comprises an intersection.

According to one embodiment, the location reference comprises exactly three pair of threads. According to other embodiments, there may be more than three pairs of threads, in particular, four, five or a larger integer number.

The threads are arranged within the hollow body such that each thread is running, preferably straight, within the hollow body and under an angle to at least one image plane of the ultrasound transducer. This is because the ultrasound image shows a slice of a three dimensional object, i.e. a slice of a patient's body part. Each thread intersecting the plane generates a dot in the ultrasound image. Two threads at an intersecting point give only one dot in the image. Therefore, if a pair of threads generates only one dot, the image plane of the ultrasound transducer is through the intersecting point.

Each pair of threads comprises a single intersection. The threads are arranged within the hollow body such that no thread lies completely within the plane of the at least three intersections. This means that only a single point of each thread lies within the plane of the three intersections, but the whole thread does not lie within this plane.

Preferably, the at least three intersections of the threads lie within a single plane, which could be called a reference plane. A plane can be defined by three points which do not lie on a straight line. Three points is the minimum number of points to define a plane. This arrangement makes it possible to define a plane which can be used to adjust an ultrasound transducer.

For the case of three intersections, only if the ultrasound plane of the ultrasound transducer also lies in the same plane which is defined by the three intersections, the ultrasound image will show only three points. In any other case, the ultrasound image will show any number between four and six points.

For a larger number of intersections than three, only if the ultrasound plane of the ultrasound transducer also lies in the same plane which is defined by the number of intersections n, the ultrasound image will show a number of points corresponding to the number of intersections n. In any other case, the ultrasound image will show any number between n + 1 and 2n.

The spatial position of the crossings may be used for the dimension calibration of the ultrasound image as well.

For the above mentioned to be true, each thread needs to comprise an ultrasound visible material. The ultrasound visible material preferably comprises nylon.

The feature that each thread runs along a straight line within the hollow body may e.g. be realized by fixing each thread at two fixing points within the hollow body, e.g. on two opposing walls.

The calibration phantom is preferably mountable to the therapy source, preferably on top of the therapy source.

Preferably there are more than three thread crossings within the calibration phantom, e.g. four or five crossings. In the case of four crossings, the shape of the crossings could be a square. In the preferred case of five crossings, the shape could be a square with the fifth crossing in the center of the square. The shape could also be like a target mark or a hair cross.

According to another embodiment when there are more than three thread crossings within the calibration phantom, the arrangement of the crossings is asymmetrical. In this case a rotations of the calibration phantom or when the calibration phantom is mounted inversely such that the arrangement of crossings is seen mirrored with respect to some plane, can be recognized easily.

Preferably, one crossing of all crossings lies within or represents the focal zone of the therapy source. In the case of five crossings, the center crossing represents the focal zone of the therapy source.

According to a second embodiment of the calibration phantom, the calibration phantom comprises a hollow body which is fillable with a liquid, in particular water, and at least three groups of threads fixed within the hollow body.

Each group of threads comprises three threads defining a z shape. Each thread of a group of threads defines a line of the z shape, wherein the three threads of a group of threads are connected consecutively. Each thread of each group of threads may be connected both to a first wall of the hollow body and a second wall of the hollow body, wherein the second wall opposes the first wall. For the second thread to be connected consecutively with the first and third thread, it may be connected with its one end to an end of the first thread and with its other end to an end of the third thread. Each thread comprises an ultrasound visible material.

According to another embodiment, the three threads of a group of threads are not connected consecutively, but rather each thread of the three threads is not connected to each other. In this case, the three threads of a single group are arranged such that the three threads combined have a shape similar to a z shape, but the three threads are not connected.

Preferably, each group of threads is plane. More preferably, each group of threads does not lie within the same plane as any of the two other groups. However, it is not forbidden that the planes of each group may intersect with a plane of another group. According to another embodiment, the planes of the groups are parallel to each other.

The threads define a z shape when seen along the plane of the ultrasound field. This means that the z shape intersects the plane of the ultrasound field three times. For three z shapes one would see nine dots in the ultrasound image.

For a single z shape, by measuring the distances between the dots in the ultrasound image and given the real arrangement of the z shape, one can calculate how the ultrasound field cuts through the z shape. By having at least three z shapes inside the phantom, it is possible to unambiguously determine the position and orientation of the intersecting plane of the ultrasound field. For the case of three z shapes, to do this determination, one needs to mark the nine intersections of the three z shapes.

Preferably, the dimensions, in particular the length of each thread and the angles between the three threads, of each of the three z shapes are different.

The advantage of the second embodiment of the calibration phantom is that using this calibration phantom no intersections have to be searched within the ultrasound image. It is only necessary to record a single ultrasound image of the calibration phantom. This image comprises the 3 z shapes which have 3 times 3, i.e. 9 dots having a special arrangement. From this arrangement it is possible to calculate how the ultrasound plane cuts through the phantom. Preferably the position and orientation of the calibration phantom with respect to the ultrasound transducer is known. In that case, one can also calculate the absolute position and orientation of the ultrasound place.

According to a third embodiment, the calibration phantom comprises a plurality of threads, preferably at least nine, arranged within the calibration phantom. This embodiment is a generalization of the other two embodiments, in particular the second embodiment.

The plurality of threads are arranged such that the position and orientation of any ultrasound plane produced by the ultrasound transducer intersecting the plurality of threads can be determined with respect to the position and orientation of the calibration phantom using a single ultrasound image produced by the ultrasound transducer. For that case, having a single ultrasound image is enough to determine the position and orientation of the calibration phantom with respect to the ultrasound field plane. Preferably, a random selection of four threads of the in total nine threads do not lie within a single plane.

The advantage of the third embodiment of the calibration phantom is that using this calibration phantom no intersections have to be searched within the ultrasound image. It is only necessary to record a single ultrasound image of the calibration phantom to determine the relative position and orientation of the ultrasound plane with respect to the position and orientation of the calibration phantom, which preferably is known.

The following refers to the first, the second, the third embodiment and other embodiments as listed above of the calibration phantom.

Preferably the hollow body is shaped such that the ultrasound transducer is mountable in a fixed position relative to the hollow body. This could be realized by a recess in a wall of the hollow body which has a similar shape as the ultrasound transducer or a mount of the ultrasound transducer. Preferably, each crossing has a different distance to the ultrasonic transducer.

The calibration phantom may e.g. comprise a fifth visual reference.

The calibration phantom preferably further comprises a receptacle for guiding the hollow body within the receptacle. The receptacle may e.g. comprise a fifth visual reference.

The receptacle of the calibration phantom is preferably mountable to the therapy source, preferably on top of the therapy source.

An embodiment, not forming part of the present invention, relates to a method for calibrating an ultrasound image of an ultrasound transducer using a calibration phantom as described above. However, this method is not executed with the second embodiment of the calibration phantom having z shaped groups of threads.

The method comprises as a first step placing the calibration phantom at a predetermined position and a predetermined orientation. Preferably the calibration phantom is placed on top of the therapy source, such that the plane of the intersections is approximately vertical. Preferably the calibration phantom is placed on top of a coupling cushion of the therapy source.

In a next step of the method, an ultrasound transducer is placed relative to the calibration phantom such that all three intersections of the threads lie within a plane of the ultrasound field produced by the ultrasound transducer. Here, the plane of the ultrasound field intersects all at least three intersections of the threads. The ultrasound transducer is preferably placed on top of the calibration phantom such that the ultrasound plane is approximately parallel to the plane of the three intersections. The ultrasound transducer is preferably positioned such that the threads can be seen in the ultrasound image.

The ultrasound image of a single pair of threads comprises two points except for when the intersection lies within the ultrasound plane. This means that if the ultrasound plane moves in a direction towards the intersection, the two points will move towards each other, while in the opposite case, the two points will move away from each other. Hence, considering all three pairs of threads, it is possible to recognize which pairs of points correspond to a single pair of threads when slightly tilting the ultrasound plane about three orthogonal axes. This could be used to automate the method for calibration such that in the end there are only three points in the ultrasound image.

It is desirable to have automatic, real-time feedback of calibration accuracy. This is possible when the ultrasound image is shown on a display in real time.

During calibration, at least three of the intersections, e.g. three of five intersection, may be marked. By knowing the relative distance between the intersections within the phantom, the two orthogonal axes of the ultrasound image may also be calibrated. Here, a distortion factor may be determined and considered during the calibration process.

After the calibration method has been executed successfully, the position of the ultrasound plane with respect to the calibration phantom is known. As the position of the threads and the distance between the threads within the calibration phantom are known, the ultrasound image may be calibrated, so that the relationship of the pixels to the known real dimensions are known.

The distance between the visual reference of the calibration phantom and the visual reference of the ultrasound transducer may be measured by the camera.

As the spatial arrangement of the ultrasound plane with respect to the calibration phantom is known, all relevant information for determining the position and orientation of the ultrasound plane with respect to the calibration phantom is known. Therefore the position and orientation of the ultrasound plane can be calculated.

This method may be performed slightly differently such that the calibration phantom is placed at a predetermined position and a predetermined orientation with respect to a therapy source. As the method as mentioned above yields the position and orientation of the ultrasound plane with respect to the calibration phantom, having the additional information about the position and orientation of the calibration phantom with respect to a therapy source, one can determine the absolute position and orientation of the ultrasound plane with respect to the therapy source.

Having this information, it is possible to either displace patient table having the ultrasound transducer mounted to it towards the therapy source such that the stone of the patient is within the focus of the therapy source or to displace the therapy source towards the patient table such that focus of the therapy source is around the stone of the patient.

A calibration procedure using a calibration phantom is usually not an easy task which can be performed every day. However, there are some methods for checking an alignment of the calibration the ultrasound plane produced by an ultrasound transducer relative to a therapy source, in particular the shock wave source, which can be performed quick and therefore every day.

A first such method can be done by attaching a marker or a reference at a known position with respect to the therapy source of the shock wave generator, and subsequently comparing the measured position of the marker with the position which corresponds to a correctly calibrated system. The marker should be visible to the ultrasound field and the measurement should be done by the ultrasound field. The difference may indicate the deviation vector which could be used to recalibrate the system. Preferably, the marker is located in a reflector or a part of the shockwave source, e.g. within the coupling water cushion.

According to another embodiment, the reference may be an electrode in the case of an electrohydraulic system, a coil or coil rim in the case of an electromagnetic system. The reference may further be the whole diameter of the reflector, a marker at the coil or a marker behind the ultrasound bellows or in the in-line window for ultrasound or X-ray. A reference may also be contained within the coupling cushion of the shockwave source, which may allow to verify the scaling of the ultrasound image. Such a daily routine may also be done by measuring a reference using the ultrasound transducer and calculating the distance between this reference and the center of the focal zone. If this value does not differ from a value which was saved in a previous check run, then the system is calibrated correctly. If the value differs, then a new value may be saved in the system.

According to another embodiment of such a method, the ultrasound field is used to probe the geometry of the therapy source, in particular the shock wave source. First the ultrasound field is generated using the ultrasound transducer, wherein a plane of the ultrasound field intersects a known structure of the therapy source, in particular at least a part of the shock wave source, in particular at least a part of a reflector and/or a coil of the shock wave source.

The known structure may be a marker inside the therapy source, where the marker can be seen on an ultrasound image. This known structure may be detected or localized inside the therapy source by sending the ultrasound field through the coupling cushion, which is preferably filled with water. The marker may be any structure inside the therapy source, which may be detectable by the ultrasound transducer. The known structure may e.g. be a part of the therapy source or the whole therapy source. Further, the known structure may e.g. be a part of the shock wave source or the whole shock wave source, in particular a part of a reflector and/or a coil of the shock wave source.

In a next step, an ultrasound image of the ultrasound field is generated.

In a further step, a deviation of the measured known structure on the ultrasound image from a known default position of the known structure on the ultrasound image is determined, in particular calculated. The known default position may e.g. be a position which was determined in a previous run, e.g. the run a day before the present alignment method. The known default position may also be a position of the known structure which is known according to the arrangement and construction of the therapy source, e.g. the position and orientation of the marker position may be known with respect to a known position of a reference marker, in particular a visual reference marker of the therapy source.

According to another embodiment, the step of determining a deviation of the measured known structure is realized by the steps described in the following.

Before or after the imaging step, a three dimensional model of the known structure of the shock wave source is generated.

Then an overlay of a geometrical feature, in particular a contour, of the known structure of the therapy source, on the ultrasound image based on the three dimensional model of the known structure and the known relationship between the ultrasound transducer and the known structure of the therapy source is generated.

The geometrical feature may e.g. be a contour. According to another embodiment, the geometrical feature may e.g. comprise a contour of a therapy source which is slightly smaller than the real therapy source and a contour of a therapy source which is slightly larger than the real therapy source. In the overlay in the ultrasound image the image of the real therapy source should be seen between the contours of the therapy sources which are slightly smaller and slightly bigger.

Slightly smaller and slightly bigger may e.g. be deviations of 2%, 3%, 5%, and 10%.

The same could be done to any component of the therapy source, e.g. the shock wave source, the reflector, or the coil of the shock wave source. The same could be done to a part of the components mentioned in the last sentence.

The smaller and bigger contours may e.g. define a tolerance range, in which the calibration is assumed to be correct.

In a next step, a deviation of the overlay of the geometrical feature of the known structure of the therapy source on the ultrasound image from a known default position of the known structure on the image of the therapy source is determined, in particular calculated. This step could be a visual control by a user or it could be calculated based on a deviation between the imaged geometrical feature and the modeled geometrical feature of the therapy source, in particular the shock wave source, in particular the reflector and the coil of the shock wave source. This deviation could be a geometrical deviation within the ultrasound image or some other measure indicating a difference between the imaged geometrical feature and the modeled geometrical feature.

### Description of Drawings

In the following the invention will be described by way of example, without limitation of the general inventive concept, on examples of embodiment with reference to the drawings.
Figure 1 shows an image of an extracorporeal shock wave lithotripsy system with an off-line ultrasound localization.
Figure 2 shows a schematic picture illustrating the relative position of the ultrasonic transducer, the therapy source and the camera.
Figure 3 shows a schematic picture illustrating how a calibration phantom 84 is arranged relative to the ultrasonic transducer 24 and the therapy source 34 when a calibration method is performed.
Figures 4 to 7 illustrate the spatial orientation of the threads inside a calibration phantom according to a first embodiment.
Figures 8 to 11 illustrate the spatial orientation of the threads inside a calibration phantom according to a second embodiment.
Figure 12 shows an extracorporeal shock wave lithotripsy system with an off-line ultrasound localization and a C-arm.

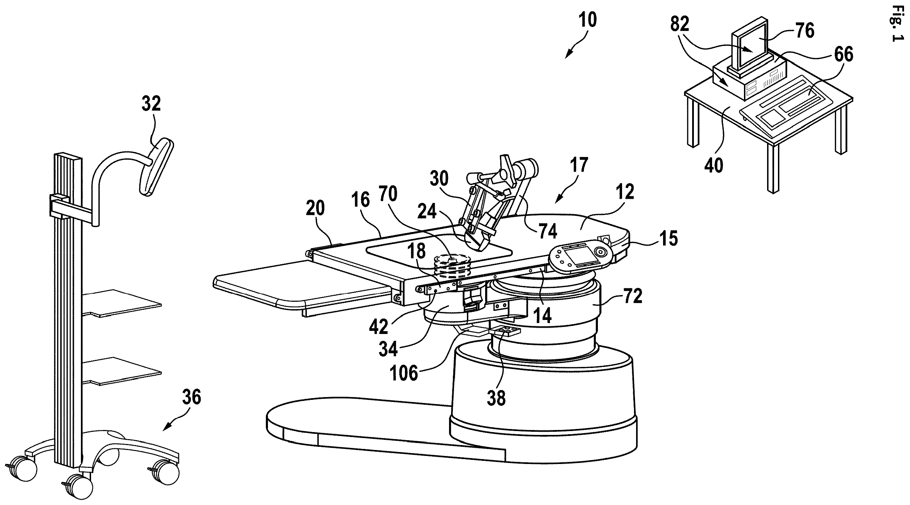

Figure 1 shows an overview image of an extracorporeal shock wave lithotripsy system 10 with an off-line ultrasound localization. In the center of the image a patient table 12 is depicted. The patient table 12 has a first mounting rail 14 shown on a first longitudinal side 15 of the patient table 12 shown at the front and a second mounting rail 16 on a second longitudinal side 17 of the patient table 12 shown at the rear. A first visual reference 18 is fixed to the first longitudinal side 15 of the patient table 12. In accordance with the present invention, the first visual reference 18 is fixed to the first mounting rail 14 of the patient table 12. In another example the first visual reference 18 is fixed to both the first longitudinal side 15 and the first mounting rail 14 of the patient table 12.

A second visual reference 20 is fixed to the second longitudinal side 17 of the patient table 12. In accordance with the present invention, the second visual reference 20 is fixed to the second mounting rail 16 of the patient table 12. In another example the second visual reference 20 is fixed to both the second longitudinal side 17 and the second mounting rail 16 of the patient table 12. For normal operation, only one of the first visual reference 18 and the second visual reference 20 is required.

An ultrasound transducer 24 having a third visual reference 30 is mounted to the second mounting rail 16 of the patient table 12 using a flexible arm 74. Alternatively the ultrasound transducer 24 may be mounted to the patient table 12 via the first mounting rail 14 or directly to the patient table 12. The third visual reference 30 may be mounted to a mounting frame of the ultrasound transducer 24.

The ultrasound transducer 24 may be used to examine a body part of the patient.

A therapy source 34 having a fourth visual reference 38 is placed below the patient table 12. The fourth visual reference 38 is placed at the end of a reference arm 106.

Each visual reference, i.e. the first visual reference 18, the second visual reference 20, the third visual reference 30, and the fourth visual reference 38 preferably has four visual reference markers 42, e.g. IR marker. The therapy source 34 comprises a shock wave source 70.

The patient table 12 may be moved relative to the therapy source 34 with the help of a displacing means 72.

A camera 32 is placed on a trolley 36 such that the first visual reference 18, the second visual reference 20, the third visual reference 30, and the fourth visual reference 38 lie within the tracking or measurement volume of the camera 32.

A measurement and evaluation system 66 having a display 76 may be located on a table 40 which is next to the patient table 12 but not within the tracking or measurement volume of the camera 32. The measurement and evaluation system 66 may e.g. be a computer. A software which runs on the computer may realize a graphical user interface 82 which is displayed on the display 76.

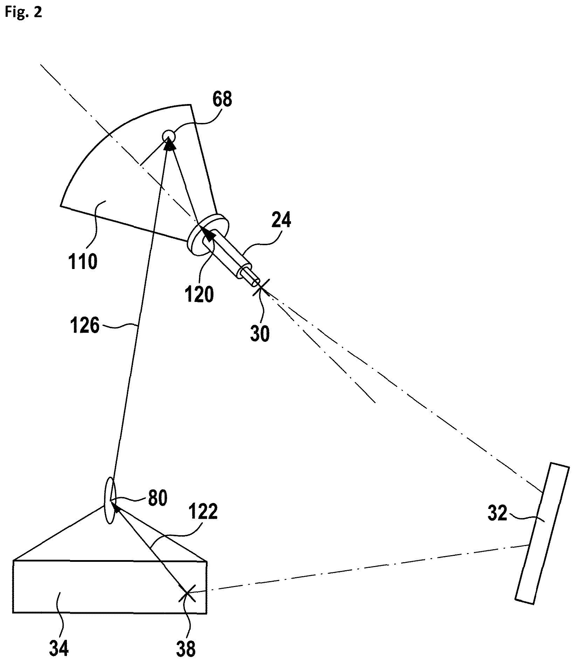

Figure 2 shows a schematic picture illustrating the relative position of the ultrasonic transducer, the therapy source and the camera.

The ultrasonic transducer 24 having the third visual reference 30 generates an ultrasound field 110. The relative position of the third visual reference 30 with respect to an origin of the ultrasound field 110 is known. This is visualized by the arrow 120 between the third visual reference 30 and a point on the ultrasonic transducer 24.

If the operator has identified an object like e.g. a stone 68 inside the patient's body, the stone 68 lies within the ultrasound field 110. Using the third visual reference 30 which is detected by camera 32 and the fact that after calibration the relative position of the stone 68 with respect to the ultrasonic transducer 24 is known, an absolute position of the stone 68 is known.

The therapy source 34 having the fourth visual reference 38 generates a focal zone 80 at a known position relative to the therapy source 34. This is visualized by the arrow 122 between the fourth visual reference 38 and the center of the focal zone 80. Using the fourth visual reference 38 which is detected by camera 32 and the fact that the relative position of the focal zone 80 with respect to the therapy source 34 is known, an absolute position of focal zone 80 is known. With this information, a difference vector between the center of the focal zone 80 and the position of the stone 68 may be calculated. This difference vector is also shown in Figure 2 as arrow 126. The difference vector could be used to move the stone 68 to the center of the focal zone 80 by moving the patient towards the therapy source 34 or vice versa by moving the therapy source 34 towards the patient.

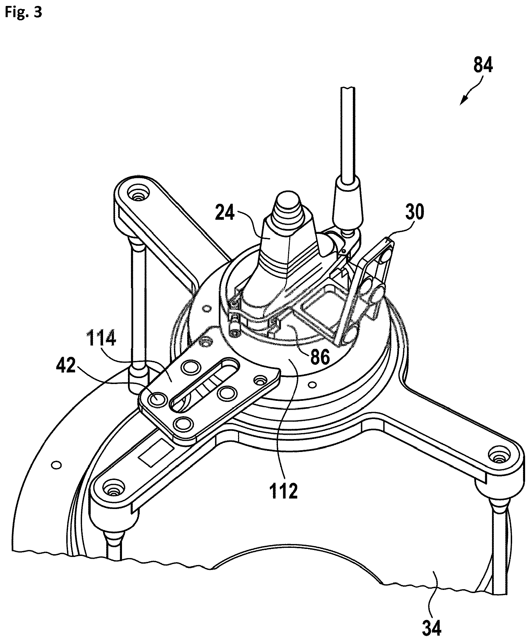

Figure 3 shows a schematic picture illustrating how a calibration phantom 84 is arranged relative to the ultrasonic transducer 24 and the therapy source 34 when a calibration method is performed.

The calibration phantom 84 comprises a hollow body 86 and a receptacle 112 into which the hollow body 86 can be guided. The head of the ultrasonic transducer 24 is placed inside the hollow body 86. Here the ultrasonic transducer may engage a recess in a wall of the hollow body 86. The hollow body 86 preferably rests on the bottom wall of the receptacle 112.

The receptacle 112 preferably has a cylindrical interior and a circumferential projection on the outside which is used to hold the receptacle 112 on a toroidal frame having three legs. The three legs may be attached to the top of the therapy source to mechanically connect the calibration phantom 86 to the therapy source 34.

The receptacle 112 further has a frame holding the fifth visual reference 114 with four visual reference markers 42.

The receptacle 112 is filled with water such that the hollow body 86 is also filled with water. The filling height of the water is such that the head of the ultrasound transducer 24 is completely immersed.

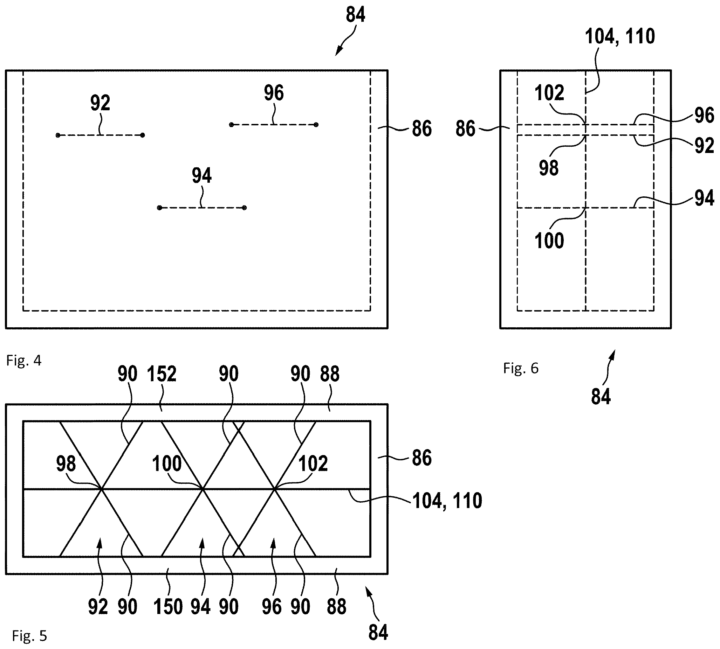

Figure 4 to 7 illustrate a first embodiment of the calibration phantom 84 of Figure 3.

In figure 4 a hollow body 86 of calibration phantom 84 is shown as seen from a side with a cut front wall. The ultrasound transducer (not shown) is attached to the top side radiating into the calibration phantom 84. Inside the hollow body 86 there are three pairs 92, 94, 96 of threads 90. The three intersections 98, 100, 102 of the three pairs 92, 94, 96 of threads 90 lie within a vertical plane 104 of the three intersections 98, 100, 102. Each thread 90 is attached to the hollow body 86, preferably to opposing walls 88 of the hollow body 86.

Figure 5 shows a top view of the calibration phantom 84 of Figure 3.

Figure 6 shows a sectional view of the calibration phantom 84 of Figure 3 seen along the long side where a side wall is cut.

According to another embodiment, on the top end of the hollow body 86 there may be on one side a recess for correctly orienting the head of the ultrasound transducer 24.

The threads 90 are attached between opposing walls 88 of the hollow body 86. It can also be seen in Figures 5 and 6 that the three intersections 98, 100, 102 do not lie on a straight line but within the plane 104. While the intersection 100 is closest to the lower side of the hollow body 86, the intersection 102 is closest to the open side of the hollow body 86. The intersection 98 lies in between the intersections 100 and 102, but is closer to the intersection 102. This corresponds to the fact that the pair 94 of threads 90 is lowest and the pair 96 of threads 90 is located closest to the opening of the hollow body 86. The pair 92 of threads 90 lies in between the pairs 94 and 96 of threads 90, but is closer to the pair 96 of threads 90, as can be seen in figure 4.

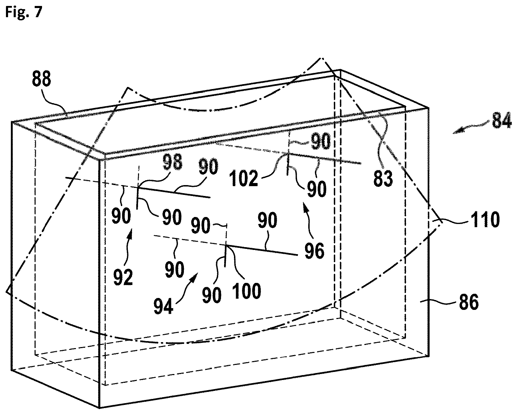

Figure 7 shows a three dimensional illustration of the calibration phantom 84 of Figure 3. Figure 7 also shows the position of the ultrasound field 110 when the ultrasound field 110 is calibrated correctly. In that case the intersections 98, 100, 102 of the three pairs 92, 94, 96 of threads 90 lie within the plane of the ultrasound field 110. In that case the plane 104 of the three intersections 98, 100, 102 of the three pairs 92, 94, 96 of threads 90 coincides with the plane of the ultrasound field 110.

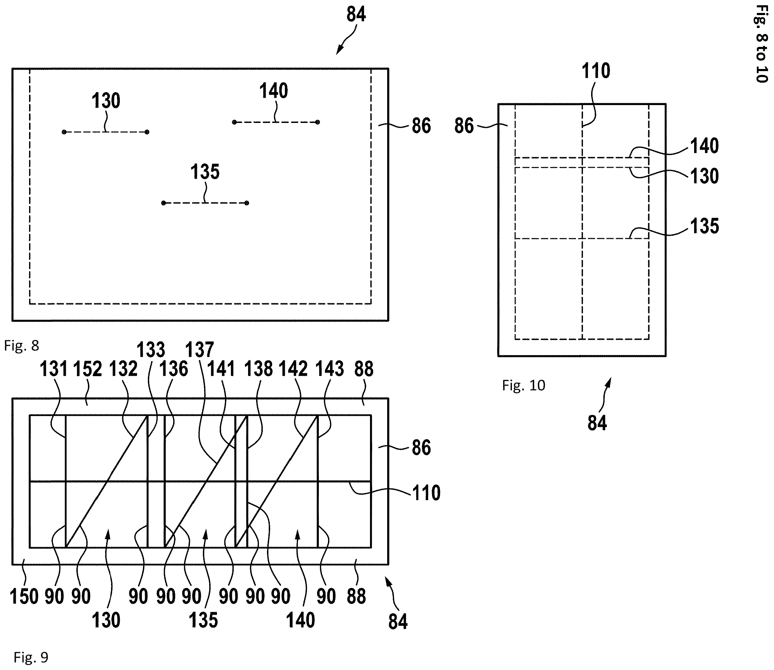

Figure 8 to 11 illustrate a second embodiment of the calibration phantom 84 of Figure 3.

In figure 8 a hollow body 86 of calibration phantom 84 is shown as seen from a side with a cut front wall.

Figure 9 shows a top view of the calibration phantom 84 of Figure 8.

Figure 10 shows a sectional view of the calibration phantom 84 of Figure 8 seen along the long side where a side wall is cut.

In figure 8 a hollow body 86 of calibration phantom 84 is shown as seen from a side with a cut front wall. The ultrasound transducer (not shown) is attached to the top side radiating into the calibration phantom 84. Inside the hollow body 86 there are three groups 130, 135, 140 of threads 90. Each thread 90 is attached to opposing walls 88 of the hollow body 86, i.e. a first wall 150 and the second wall 152. Group 140 of threads 90 is located closest to the opening of the hollow body 86, group 135 of threads 90 is located farthest from the opening of the hollow body 86. The group 130 of threads 90 is located in between the groups 135 and 140, however it is closer to the group 140 of threads 90.

Each group 130, 135, 140 of threads 90 comprises three threads 131, 132, 133, 136, 137, 138, 141, 142, 143 defining a z shape. In figure 9, you can be seen that the groups 130, 135 140 of threads 90 each has the shape of the letter z. As the groups 130, 135, 140 seen from above, the shape is an inverse or mirrored shape of the letter z. If the groups 130, 135, 140 were seen from below, the shape would be the shape of a regular letter z.

As can be seen in figure 9, group 130 comprises the threads 131, 132, 133, group 135 comprises the threads 136, 137, 138 and group 140 comprises the threads 141, 142, 143. Each thread 131, 132, 133, 136, 137, 138, 141, 142, 143 of a group 130, 135, 140 of threads defines a line of the z shape.

The three threads 131, 132, 133, 136, 137, 138, 141, 142, 143 of each group 130, 135, 140 of threads 90 are connected consecutively, wherein a first thread 131, 136, 141, a second thread 132, 137, 142 and a third thread 133, 138, 143 of each group 130, 135, 140 of threads 90 is connected both to a first wall 150 of the hollow body 86 and a second wall 152 of the hollow body 86, the second wall 152 preferably opposing the first wall 150. The second thread 132, 137, 142 is both connected with its one end to an end of the first thread 131, 136, 141 and with its other end to an end of the third thread 133, 138, 143, Each thread 90 comprises an ultrasound visible material, in particular nylon.

Figure 10 shows that the three groups 113, 135, 140 of threads 90 are parallel to each other and arranged at different distances from the ground wall of the hollow body 86. While group 140 of threads 90 is farthest from the ground wall of the hollow body 86, group 135 of threads 90 is located closest to the ground floor of the hollow body 86. The group 130 of threads 90 is located in between the groups 135 and 140, however it is closer to the group 140 of threads 90. In figure 9, the can be seen that the plane of the ultrasound field 110 intersects all of the threads 90. From the arrangement of the in total 9 intersections, it can be calculated how the calibration phantom 84 is oriented with the respect to the ultrasound field 110.

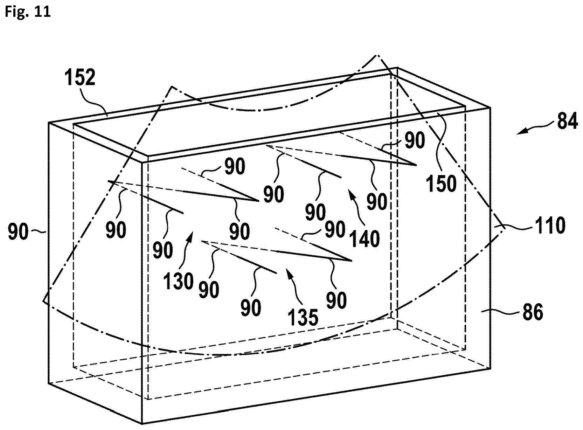

Figure 11 shows a three dimensional illustration of the second embodiment of the calibration phantom 84 of Figures 8 to 10. Figure 11 also shows the position of the ultrasound field 110 when used for calibration. The plane of the ultrasound field 110 should intersect all threads 131, 132, 133, 136, 137, 138, 141, 142, 143 of all groups 130, 135, 140 of threads 90. It can also be seen in figure 11 that each thread 131, 132, 133, 136, 137, 138, 141, 142, 143 is divided by the ultrasound field 110 into a first part which is closer to the first wall 150 and a second part which is closer to the second wall 152. The first parts are depicted using continuous or solid lines, while the second parts are depicted using dashed lines.

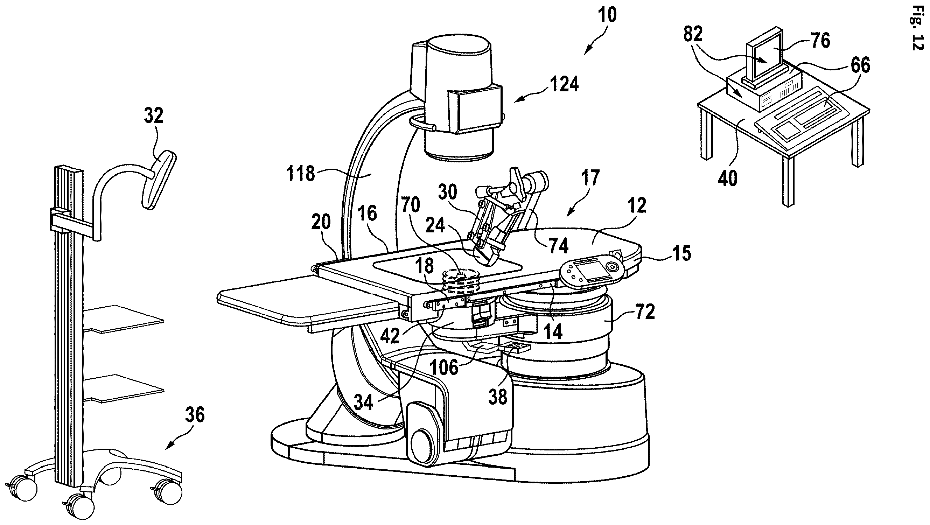

Figure 12 shows an extracorporeal shock wave lithotripsy system, similar to the system shown in figure 1, but with an off-line ultrasound localization and a C-arm X-ray device. In this embodiment, the operator should have the option to choose between the ultrasound transducer 24 and a C-arm 118 for determining the position of a stone 68. The C-arm is preferably mobile and has an X-ray source 124 for producing X-ray images of the body part of the patient. The X-ray images may preferably be high-resolution images and may preferably be produced in real time. The C-arm 118 may also comprise a visual reference, i.e. a sixth visual reference for tracking a position and orientation of the C-arm 118.

### Reference numerals

- 10: extracorporeal shock wave lithotripsy system:
- 12: patient table
- 14: first mounting rail
- 15: first longitudinal side
- 16: second mounting rail
- 17: second longitudinal side
- 18: first visual reference
- 20: second visual reference
- 24: ultrasound transducer
- 30: third visual reference
- 32: camera
- 34: therapy source
- 36: trolley
- 38: fourth visual reference
- 40: table
- 42: visual reference marker
- 66: measurement and evaluation system
- 68: stone
- 70: shock wave source
- 72: displacing means
- 74: flexible arm
- 76: display
- 80: focal zone
- 82: graphical user interface
- 84: calibration phantom
- 86: body
- 88: wall
- 90: thread
- 92: pair of threads
- 94: pair of threads
- 96: pair of threads
- 98: intersection of threads
- 100: intersection of threads
- 102: intersection of threads
- 104: plane of the three intersections
- 106: reference arm
- 110: ultrasound field
- 112: receptacle
- 114: fifth visual reference
- 118: C-arm
- 120: arrow visualizing a vector between the third visual reference and a point on the ultrasonic transducer
- 122: arrow visualizing a vector between the fourth visual reference and the center of the focal zone
- 124: X-ray source
- 126: arrow visualizing a difference vector between the center of the focal zone and the position of the stone
- 130: first group of threads
- 131: first thread of the first group of threads
- 132: second thread of the first group of threads
- 133: third thread of the first group of threads
- 135: second group of threads
- 136: second thread of the second group of threads
- 137: third thread of the second group of threads
- 140: third group of threads
- 141: first thread of the third group of threads
- 142: second thread of the third group of threads
- 143: third thread of the third group of threads
- 150: first wall of the hollow body
- 152: second wall of the hollow body

## Claims

1. Extracorporeal shock wave lithotripsy system (10) comprising:
a camera (32) for capturing images of visual references, the camera (32) being arranged on a trolley (36) which is freely movable,
a patient table (12) having a first mounting rail (14) along a first longitudinal side (15) of the patient table (12) and a second mounting rail (16) along a second longitudinal side (17) of the patient table (12), the second longitudinal side (17) being opposite to the first longitudinal side (15), a first visual reference (18) being arranged on the first mounting rail (14) or the first longitudinal side (15) and a second visual reference (20) being arranged on the second mounting rail (16) or the second longitudinal side (17),
an ultrasound transducer (24) for examining and imaging a body part comprising an object, in particular a stone (68), of a patient, the ultrasound transducer (24) having a third visual reference (30),
a therapy source (34) for treating the body part of the patient, the therapy source (34) comprising a fourth visual reference (38) and a shock wave source (70) having a focal zone (80) for destroying the stone (68),
a displacing means (72) for changing a relative position of the patient table (12) and/or the therapy source (34),
a measurement and evaluation system (66) connected to the ultrasound transducer (24), the camera (32), the therapy source (34), and the displacing means (72), the measurement and evaluation system (66) inter alia being adapted to evaluate relative positions between at least two of the ultrasound transducer (24), the therapy source (34), the first mounting rail (14) and the second mounting rail (16),
a display (76) connected to the measurement and evaluation system (66) for displaying a relative position of at least the body part and a position of the focal zone (80) of the therapy source (34)
wherein
the ultrasound transducer (24) is mountable to the patient table (12) for treatment of the patient, wherein the ultrasound transducer (24) is freely adjustable in space and lockable in a desired position and orientation
wherein
the measurement and evaluation system (66) and/or the display (76) comprise a graphical user interface (82) for inter alia displaying a positional relationship, in particular an overlay, of the focal zone (80) of the therapy source (34) and the ultrasound plane generated by the ultrasound transducer (24) wherein the graphical user interface (82) is adapted to show a detection region being the region wherein a visual reference may be detected by the camera of at least one of the first visual reference, the second visual reference, the third visual reference and the fourth visual reference, wherein the graphical user interface is adapted to show a detection region of the third visual reference and the fourth visual reference.

2. Extracorporeal shock wave lithotripsy system (10) according to claim 1, **characterized in that**
the graphical user interface (82) is adapted to show the graphical position of the ultrasound plane fixed while the graphical position of the focal zone (80) is movable relative to the graphical position of the ultrasound plane, and/or
the graphical user interface (82) is adapted to show the graphical position of the focal zone (80) fixed while the graphical position of the ultrasound plane is movable relative to the graphical position of the ultrasound plane, and/or
the graphical user interface (80) is adapted to show positions of at least two of the first visual reference, the second visual reference, the third visual reference and the fourth visual reference within a tracking volume of the camera (32).

3. Extracorporeal shock wave lithotripsy system (10) according to claim 1, **characterized in that**
each of the first visual reference (16), the second visual reference (20), the third visual reference (30) and the fourth visual reference (38) comprises three visual reference markers (42), preferably four visual reference markers (42).

4. Extracorporeal shock wave lithotripsy system (10) according to claim 1 or 2, **characterized in that**
the ultrasound transducer (24) is mountable to the patient table (12) using a flexible arm (74).

5. Extracorporeal shock wave lithotripsy system (10) according to one the preceding claims,
**characterized in that**
the therapy source (34) comprises a reference arm (106) which is rotatably arrangeable with respect to the therapy source (34), the reference arm (106) comprising the fourth visual reference (38).

6. Extracorporeal shock wave lithotripsy system (10) according to one of the preceding claims,
**characterized in that**
the displacing means (72) is adapted to change a relative position of the patient table (12) and/or the therapy source (34) such that a position of the stone (68) falls within the focal zone (80), wherein the changing of the relative position may be executed automatically or manually.

## Patentansprüche

1. Extrakorporales Stoßwellenlithotripsiesystem (10), umfassend:
eine Kamera (32) zum Aufnehmen von Bildern von visuellen Referenzen,
wobei die Kamera (32) auf einem frei beweglichen Wagen (36) angeordnet ist,
einen Patiententisch (12) mit einer ersten Montageschiene (14) entlang einer ersten Längsseite (15) des Patiententisches (12) und einer zweiten Montageschiene (16) entlang einer zweiten Längsseite (17) des Patiententisches (12), wobei die zweite Längsseite (17) der ersten Längsseite (15) gegenüberliegt, wobei eine erste visuelle Referenz (18) an der ersten Montageschiene (14) oder der ersten Längsseite (15) und eine zweite visuelle Referenz (20) an der zweiten Montageschiene (16) oder der zweiten Längsseite (17) angeordnet ist,
einen Ultraschallwandler (24) zum Untersuchen und Abbilden eines Körperteils umfassend ein Objekt, insbesondere einen Stein (68), eines Patienten,
wobei der Ultraschallwandler (24) eine dritte visuelle Referenz (30) hat,
eine Therapiequelle (34) zum Behandeln des Körperteils des Patienten, wobei die Therapiequelle (34) eine vierte visuelle Referenz (38) und eine Stoßwellenquelle (70) mit einer Fokuszone (80) zum Zerstören des Steins (68) umfasst,
ein Verschiebemittel (72) zum Verändern einer relativen Position des Patiententisches (12) und/oder der Therapiequelle (34),
ein Mess- und Auswertesystem (66), das mit dem Ultraschallwandler (24),
der Kamera (32), der Therapiequelle (34), und dem Verschiebemittel (72) verbunden ist, wobei das Mess- und Auswertesystem (66) unter anderem angepasst ist, relative Positionen zwischen mindestens zwei von dem Ultraschallwandler (24), der Therapiequelle (34), der ersten Montageschiene (14) und der zweiten Montageschiene (16) auszuwerten,
ein mit dem Mess- und Auswertesystem (66) verbundenes Display (76) zum Anzeigen einer relativen Position zumindest des Körperteils und einer Position der Fokuszone (80) der Therapiequelle (34)
wobei
der Ultraschallwandler (24) zur Behandlung des Patienten an dem Patiententisch (12) montierbar ist, wobei der Ultraschallwandler (24) im Raum frei verstellbar und in einer gewünschten Position und Ausrichtung arretierbar ist
wobei
das Mess- und Auswertesystem (66) und/oder das Display (76) eine graphische Benutzerschnittstelle (82) umfassen, um unter anderem eine Positionsbeziehung, insbesondere eine Überlagerung, der Fokuszone (80) der Therapiequelle (34) und der vom Ultraschallwandler (24) erzeugten Ultraschallebene anzuzeigen, wobei die graphische Benutzerschnittstelle (82) angepasst ist, um einen Detektionsbereich anzuzeigen, welcher der Bereich ist,
worin eine visuelle Referenz von mindestens einer von der ersten visuellen Referenz, der zweiten visuellen Referenz, der dritten visuellen Referenz und der vierten visuellen Referenz von der Kamera detektiert werden kann, wobei die graphische Benutzerschnittstelle angepasst ist, um einen Detektionsbereich der dritten der dritten visuellen Referenz und der vierten visuellen Referenz anzuzeigen.

2. Extrakorporales Stoßwellenlithotripsiesystem (10) nach Anspruch 1,
**dadurch gekennzeichnet, dass**
die graphische Benutzerschnittstelle (82) angepasst ist, um die graphische Position der Ultraschallebene fest zu zeigen, während die graphische Position der Fokuszone (80) relativ zur graphischen Position der Ultraschallebene beweglich ist,
und/oder
die graphische Benutzerschnittstelle (82) angepasst ist, um die graphische Position der Fokuszone (80) fest zu zeigen, während die graphische Position der Ultraschallebene relativ zu der graphischen Position der Ultraschallebene beweglich ist,
und/oder
die graphische Benutzerschnittstelle ( 80 ) angepasst ist, um Positionen von mindestens zwei der ersten visuellen Referenz, der zweiten visuellen Referenz, der dritten visuellen Referenz und der vierten visuellen Referenz innerhalb eines Tracking-Volumens der Kamera (32) zu zeigen.

3. Extrakorporales Stoßwellenlithotripsiesystem (10) nach Anspruch 1,
**dadurch gekennzeichnet, dass**
jede der ersten visuellen Referenzen (16), der zweiten visuellen Referenzen (20), der dritten visuellen Referenzen (30) und der vierten visuellen Referenzen (38) drei visuelle Referenzmarkierungen (42), vorzugsweise vier visuelle Referenzmarkierungen (42) umfasst.

4. Extrakorporales Stoßwellenlithotripsiesystem (10) nach Anspruch 1 oder 2, **dadurch gekennzeichnet, dass**
der Ultraschallwandler (24) unter Verwendung eines flexiblen Arms (74) am Patiententisch (12) montierbar ist.

5. Extrakorporales Stoßwellenlithotripsiesystem (10) nach einem der vorhergehenden Ansprüche,
**dadurch gekennzeichnet, dass**
die Therapiequelle (34) einen in Bezug auf die Therapiequelle (34) drehbar anordenbaren Referenzarm (106) umfasst, wobei der Referenzarm (106) die vierte visuelle Referenz (38) umfasst.

6. Extrakorporales Stoßwellenlithotripsiesystem (10) nach einem der vorhergehenden Ansprüche,
**dadurch gekennzeichnet, dass**
die Verschiebeeinrichtung (72) angepasst ist, um eine relative Position des Patiententisches (12) und/oder der Therapiequelle (34) derart zu verändern, dass eine Position des Steins (68) in die Fokuszone (80) fällt, wobei das Ändern der relativen Position automatisch oder manuell ausgeführt werden kann.

## Revendications

1. Système de lithotripsie extracorporelle par ondes de choc (10) comprenant :
une caméra (32) pour capturer des images de références visuelles, la caméra (32) étant agencée sur un chariot (36) qui est librement mobile,
une table pour patient (12) ayant un premier rail de montage (14) le long d'un premier côté longitudinal (15) de la table pour patient (12) et un second rail de montage (16) le long d'un second côté longitudinal (17) de la table pour patient (12), le second côté longitudinal (17) étant opposé au premier côté longitudinal (15), une première référence visuelle (18) étant agencée sur le premier rail de montage (14) ou le premier côté longitudinal (15) et une deuxième référence visuelle (20) étant agencée sur le second rail de montage (16) ou le second côté longitudinal (17),
un transducteur à ultrasons (24) pour examiner et imager une partie du corps comprenant un objet, en particulier un calcul (68), d'un patient, le transducteur à ultrasons (24) ayant une troisième référence visuelle (30),
une source thérapeutique (34) pour traiter la partie du corps du patient, la source thérapeutique (34) comprenant une quatrième référence visuelle (38) et une source d'ondes de choc (70) ayant une zone focale (80) pour détruire le calcul (68),
un moyen de déplacement (72) pour changer une position relative de la table pour patient (12) et/ou de la source thérapeutique (34),
un système de mesure et d'évaluation (66) connecté au transducteur à ultrasons (24), à la caméra (32), à la source thérapeutique (34), et au moyen de déplacement (72), le système de mesure et d'évaluation (66) étant entre autres adapté pour évaluer des positions relatives entre au moins deux parmi le transducteur à ultrasons (24), la source thérapeutique (34), le premier rail de montage (14) et le second rail de montage (16),
un afficheur (76) connecté au système de mesure et d'évaluation (66) pour afficher une position relative au moins de la partie du corps et une position de la zone focale (80) de la source thérapeutique (34)
dans lequel
le transducteur à ultrasons (24) peut être monté sur la table pour patient (12) pour traiter le patient, dans lequel le transducteur à ultrasons (24) peut être librement ajusté dans l'espace et verrouillé dans une position et une orientation souhaitées
dans lequel
le système de mesure et d'évaluation (66) et/ou l'afficheur (76) comprennent une interface utilisateur graphique (82) pour afficher entre autres une relation de position, en particulier un recouvrement, de la zone focale (80) de la source thérapeutique (34) et du plan d'ultrasons généré par le transducteur à ultrasons (24), dans lequel l'interface utilisateur graphique (82) est adaptée pour montrer une région de détection qui est la région dans laquelle une référence visuelle peut être détectée par la caméra d'au moins l'une parmi la première référence visuelle, la deuxième référence visuelle, la troisième référence visuelle et la quatrième référence visuelle, dans lequel l'interface utilisateur graphique est adaptée pour montrer une région de détection de la troisième référence visuelle et de la quatrième référence visuelle.

2. Système de lithotripsie extracorporelle par ondes de choc (10) selon la revendication 1,
**caractérisé en ce que**
l'interface utilisateur graphique (82) est adaptée pour montrer la position graphique du plan d'ultrasons fixe tandis que la position graphique de la zone focale (80) est mobile par rapport à la position graphique du plan d'ultrasons,
et/ou
l'interface utilisateur graphique (82) est adaptée pour montrer la position graphique de la zone focale (80) fixe tandis que la position graphique du plan d'ultrasons est mobile par rapport à la position graphique du plan d'ultrasons, et/ou
l'interface utilisateur graphique (80) est adaptée pour montrer des positions d'au moins deux parmi la première référence visuelle, la deuxième référence visuelle, la troisième référence visuelle et la quatrième référence visuelle au sein d'un volume de suivi de la caméra (32).

3. Système de lithotripsie extracorporelle par ondes de choc (10) selon la revendication 1,
**caractérisé en ce que**
chacune parmi la première référence visuelle (16), la deuxième référence visuelle (20), la troisième référence visuelle (30) et la quatrième référence visuelle (38) comprend trois repères de référence visuelle (42), de préférence quatre repères de référence visuelle (42).

4. Système de lithotripsie extracorporelle par ondes de choc (10) selon la revendication 1 ou 2,
**caractérisé en ce que**
le transducteur à ultrasons (24) peut être monté sur la table pour patient (12) à l'aide d'un bras flexible (74).

5. Système de lithotripsie extracorporelle par ondes de choc (10) selon l'une des revendications précédentes,
**caractérisé en ce que**
la source thérapeutique (34) comprend un bras de référence (106) qui peut être agencé en rotation vis-à-vis de la source thérapeutique (34), le bras de référence (106) comprenant la quatrième référence visuelle (38).

6. Système de lithotripsie extracorporelle par ondes de choc (10) selon l'une des revendications précédentes,
**caractérisé en ce que**
le moyen de déplacement (72) est adapté pour changer une position relative de la table pour patient (12) et/ou de la source thérapeutique (34) de sorte qu'une position du calcul (68) s'inscrive au sein de la zone focale (80), dans lequel le changement de la position relative peut être exécuté automatiquement ou manuellement.
